**Europäisches Patentamt**

**European Patent Office**

**Office européen des brevets**

(19)

(11) Publication number: **0 090 467**
A2

(12) # EUROPEAN PATENT APPLICATION

(21) Application number: **83200414.7**

(22) Date of filing: **24.03.83**

(51) Int. Cl.³: **A 61 N 1/36**

(30) Priority: **25.03.82 US 361978**

(43) Date of publication of application: **05.10.83**
Bulletin 83/40

(84) Designated Contracting States: **AT BE CH DE FR GB IT LI LU NL SE**

(71) Applicant: **Spaa, Walter, Trompstraat 71, NL-2518 BM The Hague (NL)**

(72) Inventor: **Spaa, Walter, Trompstraat 71, NL-2518 BM The Hague (NL)**

(74) Representative: **Urbanus, Henricus Maria, Ir. et al, c/o Vereenigde Octrooibureaux Nieuwe Parklaan 107, NL-2587 BP 's-Gravenhage (NL)**

(54) **Cardiac stimulator.**

(57) A cardiac stimulator comprising a high voltage generating circuit arrangement having a pair of output terminals and adapted to produce across said output terminals a high voltage of sufficient magnitude for causing an electric current to flow through a high impedance load circuit connected to said output terminals, said electric current having a magnitude and waveform suitable for cardiac stimulation of an area within a body. In a preferred embodiment the cardiac stimulator is adapted to transfer the electric stimulating current to the heart and/or organs to be stimulated through the high impedance current conductive path such as presented by a conventional catheter containing a conventional liquid solution, such as a saline solution, which provides a path for conducting electrical current pulses to the area to be stimulated.

One embodiment of such a high voltage generating circuit arrangement comprises a feedback control circuit which is adapted to maintain the output current flowing through said output terminals during the respective pulse interval at a substantially constant level. In another embodiment said circuit arrangement includes sensing means for producing a control signal representative of the magnitude of the load connected to said output terminals, said control signal being effective to adjust the high voltage developed across said output terminals in order to achieve the desired output current through said load.

ACTORUM AG

Title: Cardiac stimulator.

FIELD OF THE INVENTION

The invention relates to a cardiac stimulator (pacemaker). In particular, the invention relates to a cardiac stimulator adapted either to a) stimulate the heart or another organ or both these organs of a patient by means of electrical current, especially one or more electrical pulses, in an emergency situation arising, for example, during (intensive) care of this patient, or b) to temporarily vary the heart rhythm of a patient for diagnostic purposes.

BACKGROUND OF THE INVENTION

Normally, when a patient is in (intensive) care, use is made of a catheter by means of which it is possible only to transfer measuring signals, which are indicative of parameters characteristic of a physical condition of the patient, from this patient to external measuring or monitoring equipment. In the event of an unexpected emergency situation requiring the heart and/or another organ to be stimulated, a stimulating catheter must be inserted as rapidly as possible. This generally used method is not only time-wasting and cumbersome, which can have fatal consequences in an emergency situation, but also means additional risk and/or inconvenience for the patient. These objections likewise apply to the situation in which the heart rhythm is to be temporarily varied, although then the requirement of rapid insertion is less important.

The above objections may be met by using a catheter of special structure employing electrodes that are electrically connectable through metallic conductors extending in the catheter to external equipment, thus permitting the derivation of signals from the inside the heart and/or the stimulation of the heart by means of electrical pulses.

Such special catheters, however, are relatively expensive. From considerations of economy and on the basis of statistical data, it is therefore considered desirable and justifiable to normally use catheters of simpler and cheaper structure in (intensive) care.

SUMMARY OF THE INVENTION

It is an object of the present invention to eliminate the above drawbacks and render it possible to electrically stimulate the heart and/or other organs of a patient in a simple and rapid manner while using catheters of relatively simple and cheap structure. In particular, for the purpose of such transport of current use may be made of either the column of liquid present in the catheter, particularly an electrolytic solution, or a thin non-metallic film of low electrical conductivity provided on the inner wall of the catheter, or fibrous cores of a low electrical conductivity material extending in a wall of such a catheter.

Therefore it is a further object of the subject invention to provide a cardiac stimulator capable of stimulating the heart and/or other organs in a desired manner, through a high impedance current conductive path.

More in particular it is an object of this invention to provide high voltage generating circuit arrangements capable of producing high voltage signals such as required to cause electric stimulating current to flow through a high impedance current conductive path such as presented for instance by an electrolytic solution such as for instance a normal saline solution, within a conventional catheter.

A cardiac stimulator according to the invention comprises a high voltage generating circuit arrangement having a pair of output terminals and adapted to produce across said output terminals a high voltage of sufficient magnitude for causing an electric current to flow through a high impedance load circuit connected to said output terminals, said electric current having a magnitude and waveform suitable for cardiac stimulation.

More in particular a cardiac stimulator of the invention has one or all of its output terminals connected to one or more electrically conductive wires which at their opposite ends are provided with connecting means adapted to establish an electric current conductive path extending to the lumen of a catheter so that said output terminals are electrically connected to either an electrolytic solution within said lumen, or a thin non-metallic film of low electrical conductivity provided on the inner wall of the lumen, or one or more fibrous cores of a low electrical conductivity material extending in the wall of said lumen. In an alternative embodiment the stimulator is adapted to have its output, or its part containing said output connected directly, i.e. possibly without an intermediate electric wire, connected to the liquid column within the catheter.

A preferred embodiment of a cardiac stimulator according to the invention comprises a waveform signal generator which may be of conventional design, including facilities permitting an optional adjustment of the waveform signal produced by said generator at its output, the aforesaid high voltage generating circuit arrangement being provided with a control input connected to the output of said waveform signal generator.

-4-

A cardiac stimulator according to the invention is capable of performing all of the activities feasible with conventional ·· cardiac stimulators, but using a catheter that need not include metallic conductors extending therein and stimulation electrodes connected therewith.

In other words, a cardiac stimulator according to the invention is adapted to transport the electrical signals required for a desired form of cardiac stimulation via a non-metallic current path of low electrica conductivity, i.e. high electrical impedance.

The aforesaid activities are, inter alia:

a) the application of fixed frequency stimulation pulses;

b) the application of stimulation pulses only in response to a request signal produced by a detector only in the event of an atrium or a ventricle skipping a beat;

c) a combination of the activities sub a) and b);

d) the application of stimulation pulses of a frequency higher than the normal heart rhythm, if necessary in combination with facilities for automatically switching to a desired rhythm;

e) the defibrillation of an atrium and/or ventricle by the application of one or more pulses having suitable characteristics, if necessary in combination with facilities for measuring the changes resulting from such an activity, and the repetition of this procedure either on its own initiative or in response to an external command, if necessary in combination with the possibility to alter the characteristics of the respective pulse or pulses, until the desired result has been achieved;

f) cardioversion by the application of one or more pulses (so-called "burst-pacing"), which procedure may be repeated, automatically or not, until the desired effect has been reached;

g) the (temporary) application of signals for causing rhythm failures, for example for halting "re-entry" tachycardias;

h) the stimulation via the left ventricle or the left atrium, for example in emergencies during catheterization; and

i) the so-called "double-demand pacing".

A cardiac stimulator according to the invention comprises a high voltage generating circuit arrangement adapted to produce electrical pulses of such intensity and waveform that, when such a high voltage circuit is electrically connected to a high impedance as presented by a non-metallic current path as formed by a catheter which is void of any metallic conductors, the electrical current required for the desired operation is active at the desired place. The output of such an electrical high voltage generator includes a set of electrical supply leads electrically connected, on the one hand, to one or more electrodes to be placed on the patient and, on the other hand, to a metal valve or stop cock, which may be of standard design, or an electrically conductive auxiliary means adapted to be mounted on the respective catheter. Such a valve or auxiliary means is arranged for forming an electrical connection with either an electrolytic solution present in the catheter, or a thin non-metallic film of low electrical conductivity provided on the inner wall of a lumen of the catheter, or one or more fibrous cores of a low electrical conductivity material extending in the wall of a lumen of this catheter.

In addition to unipolar operation, in which only one liquid column of an electrolytic solution or one thin non-metallic film on the inner wall or one set of fibrous cores is required, also either bipolar or multipolar operation falls within the scope of the present invention. In that case, however, two or more separate liquid columns of an electrolytic solution or two or more separate thin non-metallic films on the inner wall or two or more separate sets of fibrous cores of a low electrical conductivity material are required.

An electrical high voltage circuit according to the invention may be combined with facilities suited to prevent, inter alia, firing at inappropriate moments.

In a preferred embodiment of the invention an electrical generator for causing the muscles of a body to react to an electrical stimulus is adapted to cause an electrical current in the range of 1-5 milliamps to flow through an electrically conductive liquid, for example a conventional saline solution, providing a current path to the area of the body to be stimulated. In particular, an electric generator of this type comprises an output circuit for producing a high voltage, for example in the range of 1-10 kilovolts, across an electrical load including this electrically conductive liquid. With an internal impedance of, for example, $10^6$ ohms looking into the output circuit of the electrical generator, and an overall external impedance of $10^6$ ohms for the electrical load, a voltage of 10 kilovolts produced at the output of the generator across the load causes a current of 5 milliamps to flow therethrough. In a simple embodiment the output circuit includes a transformer adapted to produce across its output winding the high voltage as required for causing the relatively low current to flow through the load.

A cardiac stimulator according to the invention comprises a high voltage generating circuit arrangement which is adapted to produce electrical high voltage pulses having such an intensity, waveform and (if necessary) repetition rate that when such pulses are applied to a catheter which is part of the electrical load of said high voltage generating circuit arrangement and having an impedance ranging from a few ohms to some megaohms, corresponding electrical current pulses which are suitable for the desired operation, are caused to flow through said load so as to be active at the desired location.

One embodiment of such a circuit arrangement comprises a feedback control circuit which is adapted to maintain the output current during the respective pulse interval at a substantially constant level. In another embodiment said circuit arrangement includes sensing means for producing a control signal representative of the load impedance, said control signal being effective to adjust the high voltage in order to achieve the desired output current.

BRIEF DESCRIPTION OF THE DRAWINGS

Fig. 1 schematically illustrates how the invention is applied to a human patient for supplying electrical current which effectively stimulates the patient's heart;

Fig. 2 illustrates on an enlarge scale a detail of Fig. 1;

Fig. 3 illustrates an embodiment of an auxiliary device which is adapted for conveniently establishing an electrical connection between one or more high-voltage wires and the internal current conduction path for one or more catheter circuits;

Fig. 4 illustrates on an enlarge scale a detail of the embodiment shown in Fig. 3;

Fig. 5 schematically illustrates an auxiliary tube for optional use in the auxiliary device shown in Fig. 4;

Fig. 6 illustrates a general block diagram of an embodiment of a high-voltage generating circuit arrangement of the invention that circuit arrangement being capable of causing electrical current to flow through a high impedance load circuit for cardiac stimulation purposes;

Fig. 7 illustrates a general block diagram of another embodiment of a high-voltage generating circuit arrangement of the invention;

Fig. 8 illustrates a general diagram of an embodiment of an output stage for use in a high voltage generating circuit arrangement of the subject invention;

Fig. 9 illustrates a more detailed circuit diagram of an embodiment of a waveform oscillator for use in a high-voltage generating circuit arrangement of the subject invention;

Fig. 10 illustrates a more detailed circuit diagram of an embodiment of a control amplifier for use in a high voltage generating circuit arrangement of the subject invention;

Fig. 11 illustrates a more detailed circuit diagram of an end stage for use in a high-voltage generating circuit arrangement of the subject invention;

Fig. 12 illustrates a circuit diagram of an embodiment of a ECG-processing circuit which can be advantageously combined with a high-voltage generating circuit arrangement of the subject invention;

Fig. 13 illustrates a more detailed circuit diagram of another embodiment of a high-voltage generating circuit arrangement of the invention;

Fig. 14 illustrates a circuit diagram of yet another embodiment of a high-voltage generating circuit arrangement of the subject invention;

Fig. 15 illustrates a circuit diagram for providing negative DC feedback which effectively reduces to substantially zero any DC current which could be enabled to flow through an external load of a high-voltage generating circuit arrangement of the subject invention;

Fig. 16 illustrates a circuit diagram of a circuit arrangement for limiting the high output voltage of a high-voltage generating circuit arrangement of the subject invention to a desired value; and

Fig. 17 shows an alternative embodiment of the circuit arrangement of Fig. 16.

DETAILED DESCRIPTION

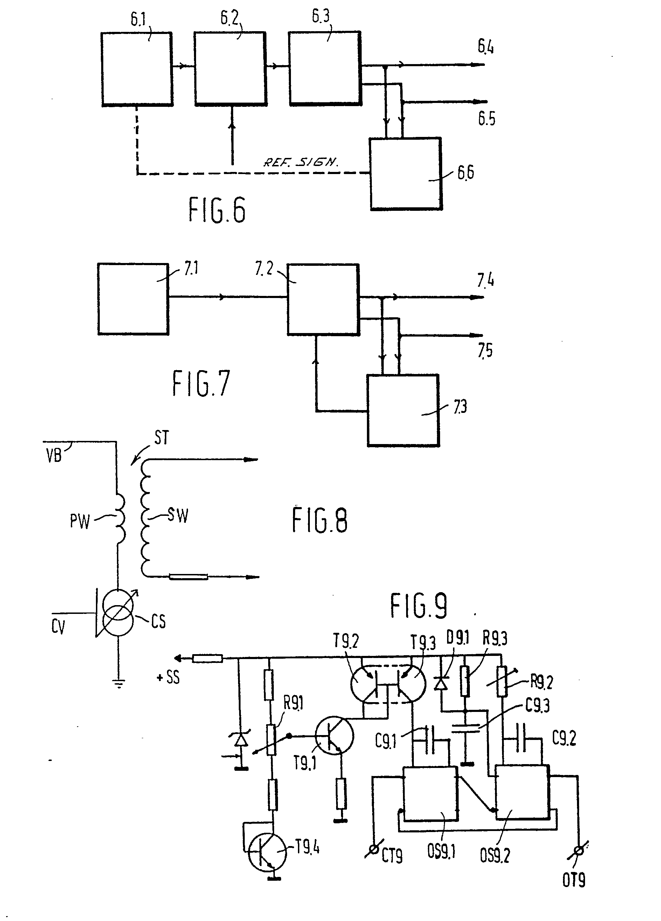

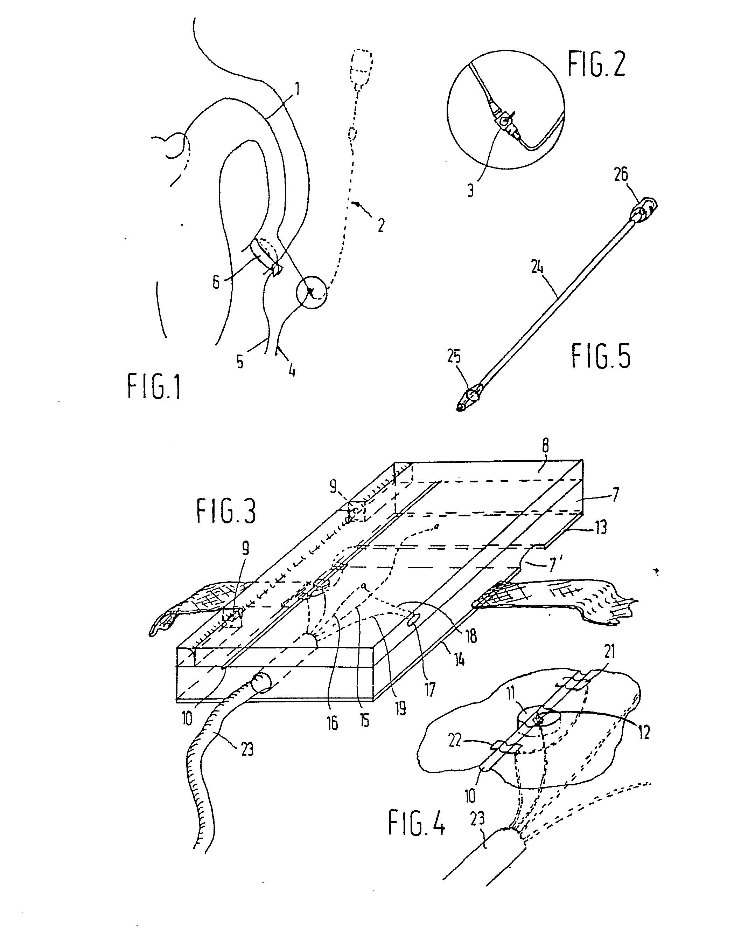

In Fig. 1 the contour of a human being and his heart to be stimulated are schematically and partly shown. The catheter 1 is of a normal and cheap structure such as used for instance for so-called drug administration purposes. This catheter normally is filled with an electrolytic solution which represents a relatively high electrical impedance for instance having a magnitude of $10^6$ ohm. This solution is supplied by a so-called drug administration system 2 which is schematically shown, and through an adjustable stop cock device 3 (see Fig. 2) of conductive material. The "hot" high voltage output wire of a high-voltage generating circuit arrangement (not shown in Fig. 1) is indicated by 4. Output wire 4 is electrically connected, by any convenient means, to stop cock 3, so that an electric current path is provided extending from wire 4 through the electrolytic solution to the desired location within the heart to be stimulated. This electric current path is completed through the body of the patient and a return wire 5 which is connected to the "patient's ground". Return wire 5 at its free end is provided with flexible connecting means 6 which are adapted to provide a detachable electrical connection with the patient's body. With the above described arrangement a high voltage pulse applied via wire 4 to stop cock 3 causes electric current to flow through the electrolytic solution within the catheter 1, by which current the heart can be effectively stimulated.

Fig. 3 illustrates an example of an auxiliary device as an alternative means for establishing an electrical connection between said output wire 4 and the electrolytic solution within catheter 1. An auxiliary device of the type as shown in Fig. 3 provides a connecting means for conveniently and rapidly establishing an electrical connection between one or more of said "hot" output wires such as 4 and the electrolytic solution within one or more catheters such as 1 each included in for instance a drug administration system (not shown in Fig. 3), and without the need to make use of a stop cock means of conductive material. The auxiliary device shown in Fig. 3 comprises a rectangularly shaped base plate 7 and a similarly shaped cover plate 8, which by means of hinges 9 is pivotally connected to base plate 7. Preferably both the base plate and the cover plate are made from a transparent, electrically insulating material. The base plate 7 is provided with one or more recesses 10 (only one is shown in Fig. 3) extending along its longest dimension for receiving a catheter therein. If desired, the cover plate of course can be provided with one or more similar recesses mating with the recesses in the base plate. The catheter extending from the front face (when looking at Fig. 3) of the auxiliary device, for instance is extending to the interior of the heart to be stimulated, while the catheter extending from the rear side (when looking at Fig. 3) of the auxiliary device, is connected to a drug administration system.

As has been shown in greater detail in Fig. 4, base plate 7 is provided with another recess spanning the groove or recess 10 in which former recess as a sealing piece 11 of silicon rubber is disposed so that an area is indicated in Fig. 4 is fluid tight closed thereby. A needle 12 of electrically conductive material is electrically connected to the respective "hot" output wire 4 and extends through base plate 7 and said sealing piece 11 into recess 10. When a catheter is disposed in recess 10 the catheter wall will be perforated by said needle 12 when cover plate 8 is brought into a position wherein it rests on the upper surface of base plate 7. In this manner the electrolytic solution within the catheter can be conveniently and rapidly connected to needle 12 and wire 4, to provide an electric current path to the interior of the heart to be stimulated.

Base plate 7 at its bottom surface (when looking at Fig. 3) is provided with a first body electrode 13 and a second body electrode 14. These electrodes are electrically insulated from each other by means of a groove 7' formed in the bottom surface of base plate 7. Groove 7' ensures that the insulation between electrodes 13 and 14 is maintained even if use is made of highly conductive paste on the patient's body. Electrodes 13 and 14 respectively have individual electric wires 15 and 16 connected thereto so as to provide a sensing circuit which can be included in a sensing device for checking whether the appropriate electric current path through the patient's skin and between these electrodes 13 and 14 has been established. As an additional option cover plate 8 is provided with a sensor 17 which through individual wires 18 and 19 can be included in the sensing device for checking whether cover plate 8 appropriately lies on base plate 7 thereby ensuring that the repective catheter is effectively perforated by its associated needle such as 12. The above described auxiliary device as a whole can be attached to a patient's body for instance by means of flexible bandage 20. By means of the above described sensing electrodes 13 and 14 and the optional sensor 17 checking circuits can be established for enabling the high voltage generating circuit arrangement. If one of these checking circuits is not appropriately established the high voltage generating circuit arrangement is disabled and therefore is not capable of producing any high voltage output. As a further option on opposite sides of

0090467

sealing piece 11 there are provided two further sensing electrodes 21 and 22 which through individual wires provide a fluid leakage detecting circuit which is included in a detector which produces a warning signal if due to leakage of the catheter a current conductive path is established between needle 12 and sensing electrodes 21 and 22 for transferring the pulses on output wire 4 to said detector. All the afore mentioned wires are included in a cable 23 which leads to the high voltage generating circuit arrangements and the respective sensing devices and leakage detector respectively.

Fig. 5 illustrates an auxiliary tube section 24 which can be optionally used with the auxiliary device shown in Figs. 3 and 4. Tube section 24 at its opposite ends is provided with connecting means 25 and 26 respectively for making a connection to a catheter leading to the heart to be stimulated and to a catheter or a tube leading to for instance the drug administration system. Tube section 24 has such a length dimension that when disposed in recess 10 of base plate 7 of the auxiliary device shown in Fig. 3 the respective connecting means 25 and 26 are extending from the respective end surfaces of the auxiliary device shown in Fig. 3. With tube section 24 disposed in recess 10 closure of cover plate 8 causes needle 12 to perforate tube 24 thereby establishing an electric connection with the electrolytic solution which is within tube section 24. After use tube 24 can be disconnected from the respective catheter ends and can be replaced by another similar tube section for further use. In this manner a considerable saving is achieved because it is not the catheter but the auxiliary tube 24 which is perforated so that the catheter can be used repeatedly and only the tube section 24 has to be replaced. Preferably such auxiliary tube is made of self sealing material.

0090467

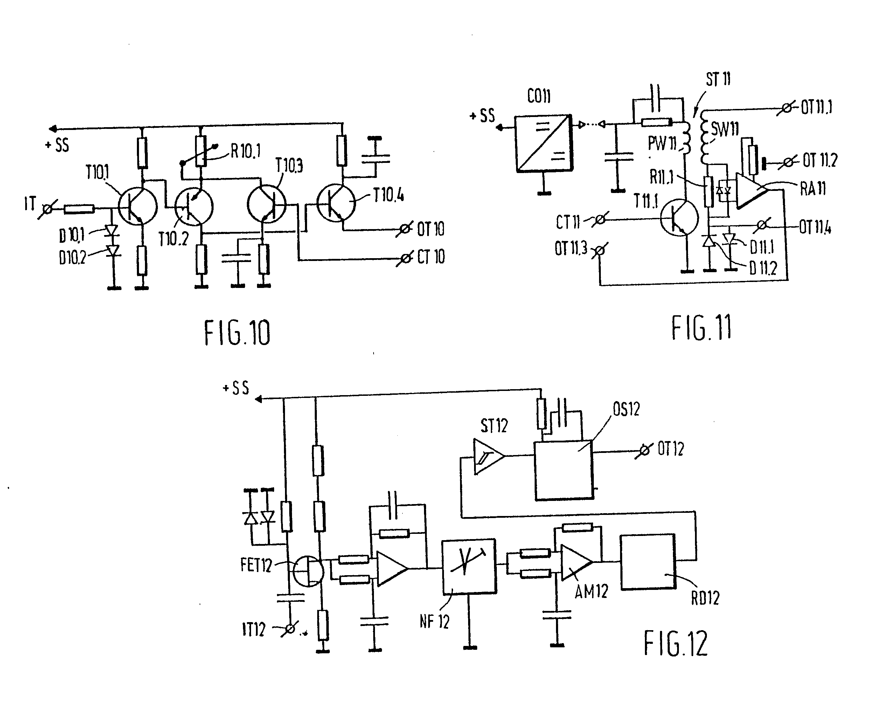

Fig. 6 illustrates an embodiment of a high voltage generating circuit arrangement of the subject invention. Such a circuit arrangement is designed for delivering a high voltage of appropriate magnitude across a high impedance load, which substantially exceeds a load such as commonly is presented to conventional cardiac stimulator devices. More in particular a load as employed in the environment of the subject invention can have a magnitude as high as for instance $10^6$ ohm. Such a load for instance is presented by a current flow path having a column of an electrolytic solution, for instance a saline solution, included therein. In other words the high voltage generating circuit arrangement is capable of generating a high voltage of such high a magnitude and waveform that an electric current is caused to flow through said high impedance load, which is sufficient and appropriate for effectively stimulating the heart or other organ in a desired manner. The embodiment shown in Fig. 6 comprises a waveform generator 6.1 which is adapted to selectively generate the various types of waveforms such as required for the desired type of stimulation; a control amplifier 6.2 for providing at its output a control voltage; an end stage 6.3 for producing in response to the control voltage applied to its input, the high voltage across its output terminals 6.4 and 6.5 respectively adapted for connection to the load (not shown) and which voltage is required for causing the desired current to flow through said load; and a sensing device 6.6 which in response to sensed output current from end stage 6.3 produces a reference signal for control amplifier 6.2. Moreover sensing circuit optionally has included therein circuitry for producing an initiating signal for instance representative of a peak in a detected R-wave for starting waveform generator 6.1 to operate in case "on demand pacing" is required. Because this is an optional feature the connection between sensing circuit 6.6 and the waveform generator 6.1 is depicted as a dashed line.

Waveform generator 6.1 can be of any conventional type such as normally used for cardiac stimulation purposes. A preferred embodiment of such a waveform generator will be discussed later herein with reference to Fig. 9. At option waveform generator 6.1 is employed as an external unit relative to the components 6.2; 6.3 and 6.6; as an alternative, waveform generator 6.1 together with the components 6.2, 6.3 and 6.6 constitutes a unitary device. Control amplifier 6.2 is adapted to process, for instance to compare, the waveform signal applied to its one input from waveform generator 6.1, and said reference signal applied to its other input, so as to provide a control signal which drives the end stage 6.3. As has been schematically shown in Fig. 8 end stage 6.3 comprises a controllable current source CS having a control input CV, and having its main current path connected in series with the primary winding PW of a step up transformer ST and an appropriate DC supply source (not shown) providing an appropriate DC supply voltage VB of for instance 400 Volt. The control voltage supplied to control input CV not only drives the end stage to produce the high output voltage across the secundary winding SW of transformer ST with the required magnitude, but in addition is effective to maintain the amplitude level of the output current pulse(s) substantially constant during occurrence thereof. By selection of an appropriate step up ratio, for instance 1:20 the high voltage necessary for causing a desired current to flow through the load connected across the secundary winding SW, is achieved when an appropriate control voltage is applied to control input CV. As an example the end stage is capable of producing for instance one or more current pulses each having a duration of about 0,5 ms in sequence with a magnitude of for instance 1 ma in a high impedance load of for instance 1 Mohm.

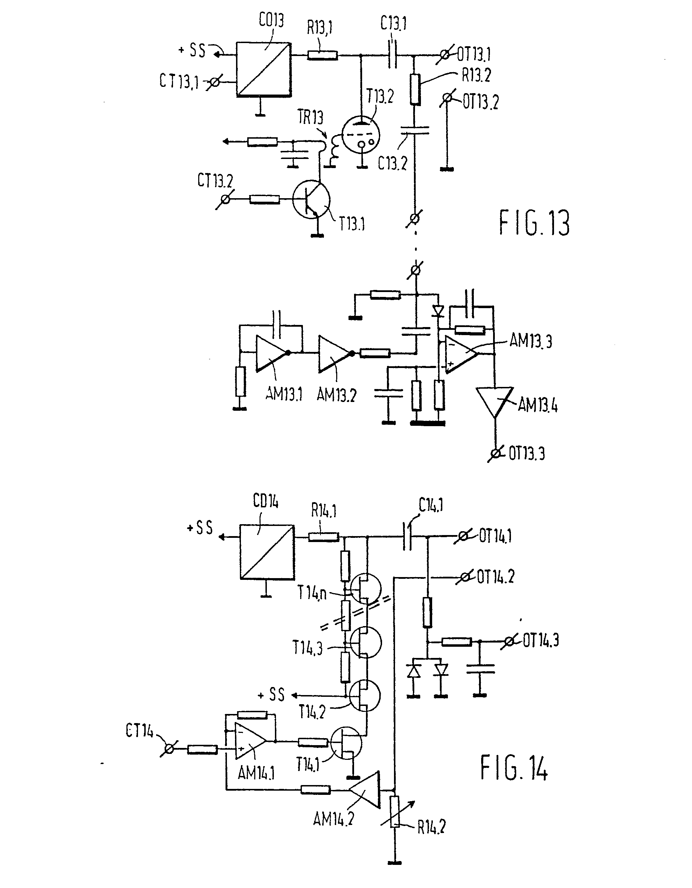

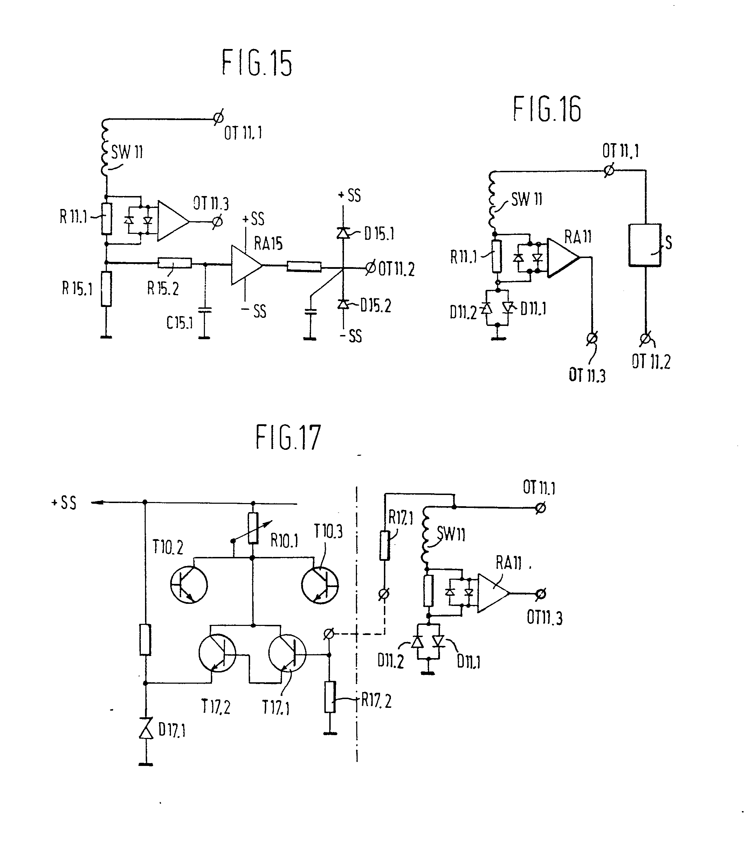

Fig. 7 is illustrative of another embodiment of a high voltage generating circuit arrangement suitable of producing electric current with the required magnitude in a high impedance load connected across its output terminals 7.4 and 7.5 respectively. The embodiment shown in Fig. 7 comprises a waveform generator 7.1 which is of a similar design as waveform generator 6.1 of Fig. 6. The output signal of generator 7.1 directly controls end stage 7.2. In this embodiment end stage 7.2 either is of a type as shown in Fig. 8 or is transformerless. Such a transformerless end stage comprises an energy storage capacitor which is charged by an appropriate source to the required high voltage. Said storage capacitor then is selectively discharged by means of a switching device which is responsive to a controlling waveform signal. As will be discussed more in detail further herein with reference to Figs. 13 and 14 sensing circuit 7.3 either is adapted to provide a control signal representative of the magnitude of the load connected across the output terminals 7.4 and 7.5, or to produce a control signal representative of the output current flowing through said load. Also in the general embodiment as shown in Fig. 7 waveform generator 7.1 either is a separate unit connectable to end stage 7.2, or is included in a unitary device together with the components 7.2 and 7.3.

In order to limit current surges due to excessive discharge currents, which for instance in a fault condition are caused to flow through the electric capacitance of the electric cable connecting the catheter to the output side a high voltage generating circuit arranged of the type described above, it is preferred to include two electric resistors in series relation, having for instance a resistance of
50 k$\Omega$ each, in the electric path connecting the "active" output terminal
of said high voltage generating circuit arrangement and the active
catheter electrode. More in particular one of these resistors is
connected between said active output terminal and the adjacent end
of the cable wire leading to the active catheter electrode, while
the other one of said resistors is connected between the opposite
end of said cable wire and said catheter electrode.

As a further alternative, the cable, more in particular one
or both cable wires, connecting the high voltage generating circuit
arrangement and the catheter, can be made of material having a certain
electrical resistance. A cable providing an electrical resistance of
for instance 50-100 k$\Omega$ can be advantageously employed, with or without
the above resistors.

Fig. 9 is illustrative of a preferred embodiment of a
waveform generator such as 6.1 shown in Fig. 6. As an advantageous
feature of this preferred embodiment the setting of a rate adjusting
potentiometer R9.1 is linearly related to the repetition rate of the
waveform signal pulses produced across the output terminal OT9
and the ground terminal of an appropriate DC supply source +SS.
In Figs. 9-14 this ground terminal is schematically depicted as
a short horizontal thickly drawn line. The waveform generator
comprises a rate determining circuit including one-shot multivibrator
OS9.1 and a RC-timing network including capacitor C9.1.

The resistive portion of said timing network comprises transistors T9.1, T9.2 and T9.3 which together with variable resistor R9.1 provide a rate determining variable resistance. The pulse width is determined by variable resistor R9.2 and capacitor C9.2 which are connected to a second one-shot multivibrator OS9.2. A starting network comprising diode D9.1, resistor R9.3 and capacitor C9.3 ensures appropriate pulse waveform and phasing irrespective of the instant at which the generator supply voltage +SS is switched on. In as long as any timing signal is absent on the control input CT of this waveform generator, at the output OT9 there are produced substantially rectangular pulses having their repetition rate determined by the setting of resistor R9.1 and their width determined by the setting of resistor R9.2. However, if an appropriate timing pulse is applied to control input CT occurring in coincidence with the time at which normally a pulse should be produced at the output OT9, the waveform generator is disabled and therefore fails to produce an output pulse at the output terminal OT9. Therefore control inputs CT is effectively used in case "on demand pacing" is desired. Such timing pulses are provided by any suitable device, for instance a blood pressure monitor, or an ECG-circuit arrangement which will be described more in detail later herein with reference to Fig. 12. Advantageously transistors T9.1, T9,2, T9.3 and T9.4 are disposed in one and same housing so as to improve the temperature stability of the waveform generator.

Fig. 10 shows an embodiment of an adjustable control amplifier such as 6.2 of the circuit arrangement shown in Fig. 6. The control amplifier comprises an input stage with transistor T10.1 and series connected diodes D10.1 and D10.2 which effectively limit pulses such as provided by an appropriate waveform generator such as 6.1 shown in Fig. 6, to its input terminal IT to an appropriate level. The input waveform signal thus limited is applied to the base electrode of a transistor T10.2 having its collector collected to the base electrode of transistor T10.3 which together with transistor T10.4 forms a differential amplifying stage. The magnitude of the waveform signal developed at the collector of transistor T10.2 can be selectively adjusted by means of potentiometer R10.1. The afore-said reference signal such as provided by sensing circuit 6.6 such as shown in Fig. 6, is provided to the base electrode of transistor T10.4 from control input terminal CT10. The control amplifier thus described provides at its output terminal OT10 a control signal which is proportional to the difference between the waveform signal applied to input terminal IT and the reference signal applied to control terminal CT10. As mentioned earlier herein said control signal drives and controls respectively the end stage such as 6.3 shown in Fig. 6.

Fig. 11 shows a more detailed diagram of an end stage of the type shown in Fig. 8. The afore mentioned DC supply voltage of for instance 400 Volt is provided by a conventional DC-DC converter CO11 which also delivers the supply voltage +SS for the other circuitry. In this example +SS is a DC-voltage of 6 Volt. Connected in series with the primary winding PW11 of step up transformer ST11 is a control transistor T11.1 which at its base electrode receives said control signal from control terminal CT11. Preferably transistor T11.1 operates in such a way that across the secondary winding SW11 of step up transformer ST11 an appropriate high voltage is developed corresponding to the control signal applied to control terminal CT11. The output terminals OT11.1 and OT11.2 respectively of this end stage are connected to said output wire 4 and said return wire 5 (see Fig. 1) for delivering the desired stimulation current at the heart or organ to be stimulated. Connected in series with the secundary winding SW11 is a control resistor R11.1. The voltage developed across control resistor R11.1 is applied to the input of reference signal amplifier RA11 which at its output produces the afore-mentioned reference signal which is representative of the output current flowing through the load connected to the output terminals OT11.1 and OT11.2. Said reference signal is delivered at the output terminal OT11.3 for connection for instance to control terminal CT10 of the control amplifier shown in Fig. 10. A variable impedance circuit comprising diodes D11.1 and D11.2 is connected in series with control resistor RA11 in order to provide a voltage representative of information regarding the activity of the heart such as for instance P-Q-R-S-T-complexes, at the output terminal OT11.4. Said signal produced at output terminal OT11.4 can be applied to the input of an ECG-processing circuit arrangement which will be discussed further herein with reference to Fig. 12.

Fig. 12 shows an embodiment illustrative of a ECG processing circuit which receives the afore mentioned signal from output terminal OT11.4 at its input terminal IT12. The ECG processing circuit arrangement has a very high input impedance provided by field-effect transistor FET 12 which receives said input signal at its gate. A notch filter NF12 which is tuned to the fundamental main's frequency filters out undesired hum components from the heart activity signal which then through an amplifier AM12 is applied to the input of a conventional R-wave detector RT12. The R-wave signal produced at the output of RD12 is through a Schmitt-trigger device ST12 applied to one-shot multivibrator OS12 which at its output produces a sequence of R-wave peaks at the output terminal OT12. The signal produced at the output OT12 can be applied to the control input CT9 in case "on demand pacing" is required as has been discussed earlier herein with reference to Fig. 9.

Figs. 13 and 14 which will be discussed in the following are illustrative of embodiments of a transformerless end stage wherein the electric current for stimulating the heart or other organ through a high impedance current path is delivered by means of an appropriately dimensioned capacitor C which is charged to the desired high voltage such as for instance 8 kilovolt. In the embodiment shown in Fig. 13 use is made of a conventional controllable DC-DC converter CO13 which delivers the DC supply voltage +SS necessary for energizing other electronic circuitry at the one hand, and a variable high voltage which can be controlled in dependence of a control signal applied to its control input terminal CT13.1 to charge capacitor C13.1 via charging resistor R13.1 at the other hand. A waveform signal such as for instance provided by a waveform generator such as for instance has been discussed with reference to Figs. 7 and 9 is applied as a control signal to control input terminal CT13.2. Such a control signal, more in particular a control pulse causes input transistor T13.1 to conduct which causes through transformer TR13 a control signal to occur at the control electrode of an electronic switching device T13.2. Consequently this electronic switching device is made conducting in response to a control pulse applied to said control input terminal CT13.2, so that capacitor C13.1 is discharged through the main current path of electronic switching device T13.2. If the output terminals OT13.1 and OT13.2 as has been discussed earlier herein with reference to Fig. 11 are connected to a high impedance current flow path, consequently a current pulse is caused to flow therein which through the fluid column of the catheter included in the load, is effective to stimulate the heart or other organ in the desired manner. With the embodiment shown in Fig. 13 each current pulse thus produced has its magnitude determined by the high voltage value as present across capacitor C13.1 at the instant at which the electronic switching device T13.2 is switched into its conductive state. In order to appropriately control the DC-DC converter CO13 the end stage of this embodiment has associated therewith a sensing circuit which is adapted to provide a control signal which is representative of the impedance value of the load connected to its output terminals OT13.1 and OT13.2. To this end the associated sensing circuitry comprises an oscillator having two cascaded amplifiers AM13.1 and AM13.2 included therein for delivering through a RC network comprising resistor R13.2 and capacitor C13.2 an appropriate waveform signal to the load connected to output terminals OT13.1 and OT13.2. A voltage representative of the load connected to said output terminals OT13.1 and OT13.2 is applied to the inverting input of an amplifier AM13.3 which is adapted to integrate said input signal. The integrated signal produced at the output of amplifier AM13.3 is applied to the input of an inverting amplifier AM13.4 which at its output terminal OT13.3 provides a control voltage for controlling said DC-DC converter CO13 via its control input CT13.1. Said control signal therefore is representative of the impedance as measured by the sensing circuitry described above so that converter CO13 delivers a high voltage for charging capacitor C13.1 to a high voltage value corresponding to the impedance of the load. In a preferred embodiment a variable impedance comprising two anti-parallel connected diodes or Zener diodes is included in series with capacitor C13.1, so as to separate the source impedance from the external load. Thereby the load impedance is measured with an improved accuracy.

In the embodiment shown in Fig. 14 use is made of a DC-DC converter CO14 for delivering a substantially constant high voltage of for instance 8 kilovolt at its one output. Also this converter CO14 is of conventional design. Therefore with this embodiment capacitor C14.1 through resistor R14.1 is charged to a substantially constant high value as necessary for causing a current to flow through the high impedance load connected to output terminals OT14.1 and OT14.2 having the desired value. This output current is caused to flow in response to a waveform signal which is provided for instance by a waveform generator such as for instance has been discussed earlier herin with reference to Fig. 7 and which waveform signal is applied to control input terminal CT14. Such waveform signal through a differential amplifier AM14.1 is effective to switch a set of cascaded field-effect transistors T14.1 - T14.n into their conductive state, so that capacitor C14.1 is discharged through these transistors causing the desired current to flow through the load connected to the output terminals OT14.1 and OT14.2. With this embodiment the magnitude of each output current pulse is measured during its occurrence by means of an output current sensing circuit comprising an inverting amplifier AM14.2 and a variable resistor R14.2. The voltage developed across said resistor R14.2 is representative of the magnitude of an output current pulse and which voltage after having been inverted by amplifier AM14.2 is applied to the inverting input of differential amplifier AM14.1. In this manner the level of an output current pulse produced in the high impedance load connected to the output terminals OT14.1 and OT14.2 is maintained at a substantial constant value during its occurrence. Similar to the embodiment shown in Fig. 11 this embodiment also includes circuitry connected to the output terminal OT14.1 for providing at the output terminal OT14.3 an ECG-signal.

In a similar manner as has been discussed with reference to Figs. 11 and 9 the ECG-signal thus produced can be effectively used in case "on demand pacing" is desired. By connecting control input terminal CT13.2 (Fig. 13) and CT14 (Fig. 14) respectively through a resistor of 500 ohm to ground, any conventional cardiac stimulator can be employed as a source for applying the afore mentioned waveform signal as a control signal for the end stage.

While specific embodiments of the invention have been disclosed, variations in structural detail within the scope of the appended claims, are possible and are contemplated. There is no intention of limitation to what is contained in the Abstract or the exact disclosure as presented herein. The above-described arrangements are merely illustrative of the application of the principles of the subject invention. Many other arrangements may be deviced by those skilled in the art without departing from the spirit and scope of this invention. These departures include other external control and/or timing devices for the control and/or timing of the respective circuits. Further all kinds of auxiliary circuits for further improving the convenience in use can be added, such as (isolated) in- or output circuits, display circuits, safety circuits, etc.

With reference to Figs. 15, 16 and 17 in the following some additional embodiments of this invention for further improving the performance thereof will be discussed. The embodiments shown in these Figs. 15-17 are related to the embodiments shown in Figs. 10 and 11 and therefore the same parts have been provided with the same reference symbols. It has been found that with a circuit arrangement of Figs. 10 and 11 an undesired DC current component is caused to flow through the output circuit connected to the secondary transformer winding SW11 if for instance use is made of electrodes of different materials. In principle such undesired DC current can be inhibited by connecting a capacitor in series with said secondary winding SW for blocking any DC current from flowing through the load connected to the output terminals OT11.1 and OT11.2. However, due to the rather stringent characteristics which are required for such a capacitor preference is given to the embodiment shown in Fig. 15. As shown therein an additional resistor R15.1 is connected in series with resistor R11.1. Across resistor R15.1 a voltage is developed representative of the undesired DC current which is to be eliminated. This voltage is applied to an integrating network comprising resistor R15.2 and capacitor C15.1 and having a relatively long time constant. The voltage thus integrated then is applied to an inverting amplifier RA15 which receives its supply voltage from the supply terminals +SS and -SS. The amplifier output including output terminal OT11.2 is connected to the "non-active" patient's electrode. By means of clamping diodes D15.1 and D15.2 said output is symmetrically connected with respect to the supply source +SS and -SS. With the above described circuit arrangement the electrical circuit including the patient's body is subjected to a negative feedback operation whereby the resulting DC current through the output circuit is reduced to substantially zero. In this manner an efficient compensation is obtained for any undesired DC current which possibly might flow in the load circuit connected to output terminal OT11.1. In an alternative embodiment the amplifier RA15 can be avoided, thereby that the integrating network R15.2; C15.1 is connected to the output of amplifier RA11. The output of the integrating network then is connected to the output terminal OT11.2.

If no external load is connected to the output terminals OT11.1 and OT11.2 a very high voltage can develop across these output terminals due to the absence of any output current which opposes the build-up of such high output voltage. Such high output voltage under no-load condition is undesired because components such as transformers and other parts can be damaged thereby. The circuit arrangement schematically shown in Fig. 16 is a first embodiment for limiting the high voltage across output terminals OT11.1 and OT11.2 under the no-load condition. Connected across these output terminals is a voltage dependent element, such as for instance a voltage dependent resistor or a semiconductor element S. This element S is switched into its low-ohmic state if the voltage developed there across exceeds a predetermined threshold value. Thereby the current which normally flows through the patient is simulated. Consequently the feedback action through amplifier RA11 is effective to reduce the output voltage to approximately the predetermined threshold value of element S. The magnitude of this threshold is determined by the design characteristics of element S. Once the voltage across output terminals OT11.1 and OT11.2 has reached a value lower than the threshold said element is switched into its high-ohmic condition again.

An alternative embodiment is shown in Fig. 17. This embodiment relies on a combination of the circuit arrangements shown in Figs. 10 and 11. Two resistors R17.1 and R17.2 which are series connected to each other between the output terminal OT11.1 and circuit's mass, constitute  a voltage divider whereby the relatively high output voltage is reduced to an appropriate low value voltage across resistor R17.2. This voltage is applied to an amplifier comprising for instance a Darlington pair of transistors T17.1 and T17.2 which are connected in series with a Zener diode D17.1 to circuit's mass. As soon as the voltage developed across resistor R17.2 becomes higher than the overall voltage across Zener diode D17.1 and the transistors T17.2 and T17.1 connected in series therewith, through the collector circuit thereof a current is caused to flow by which a voltage limiting action with respect to the output voltage across terminals OT11.1 and OT11.2 is obtained. The embodiment of Fig. 17 is to be preferred compared to the embodiment of Fig. 16, because in the former there is no need for the use of a voltage dependent element such as element S which from the technical point of view is less practical.

0090467

C L A I M S

1.    A cardiac stimulator comprising a high voltage generator circuit arrangement having a pair of output terminals and adapted to produce across said output terminals a high voltage of sufficient magnitude for causing an electric current to flow through a high impedance load circuit connected to said output terminals having a magnitude and waveform suitable for cardiac stimulation.

2.    A cardiac stimulator according to claim 1, wherein one of said output terminals is connected to an electrically conductive wire which at its opposite end is provided with connecting means adapted to establish an electric current conductive path extending to the lumen of a catheter.

3.    A cardiac stimulator according to claim 2, wherein said connecting means comprises a needle adapted to perforate a wall portion of said catheter.

4.    A cardiac stimulator according to claim 2, wherein said connecting means comprises a needle adapted to perforate a tube section having at its opposite ends auxiliary connecting means whereby said tube section is connectable to a catheter flow circuit.

5.    A cardiac stimulator according to claim 3, wherein said needle is disposed in a base plate means and extends into a groove extending in one main surface of said base plate means, said groove being dimensioned for receiving a section of a catheter therein; said base plate means having pivotally connected thereto a cover portion which is disposed opposite to said one main surface.

6.    A cardiac stimulator according to claim 4, wherein said needle is disposed in a base plate means having a groove extending in one main surface thereof formed therein said groove being dimensioned for receiving said tube section; said base plate means having pivotally connected thereto a cover portion which is disposed opposite to said one main surface.

7.    Tube section having at its opposite ends auxiliary connecting means for connecting said tube section into a catheter flow circuit, said tube section being intended for use in combination with a cardiac stimulator according to claim 4.

8.    A cardiac stimulator according to claim 2, wherein said connecting means comprises a male connector adapted for insertion into a branch portion of a conventionally shaped stop cock of conductive material.

9.    A cardiac stimulator according to claim 2, wherein said connecting means are adapted to establish at least two electric current conductive paths extending to the lumen of a catheter.

10.    A cardiac stimulator according to claim 2, wherein said connecting means are adapted to provide an electric current conductive path to a thin, non-metallic film on the inner wall of a catheter.

11.    A cardiac stimulator according to claim 2, wherein said connecting means are adapted to provide an electric current conductive path to one or more fibrous non-metallic current paths in a catheter.

12.    A cardiac stimulator comprising a high voltage generating circuit arrangement having a pair of output terminals, voltage generating means adapted to produce across said output terminals a high voltage of sufficient magnitude for causing an electric current to flow through a high impedance load connected to said output terminals said electric current having a magnitude and waveform suitable for cardiac stimulation, and control means which in response to a control signal are effective to control said high voltage developed across said output terminals.

13.     A cardiac stimulator comprising a high voltage generating circuit arrangement having a pair of output terminals, voltage generating means adapted to produce across said output terminals a high voltage of sufficient magnitude for causing an electric current to flow through a high impedance load circuit connected to said output terminals, said electric current having a magnitude and waveform suitable for cardiac stimulation, control means which in response to a control signal are effective to control said high voltage developed across said output terminals, and a sensing circuit connected to one of said output terminals and adapted to produce said control signal.

14.     A cardiac stimulator according to claim 13, wherein said sensing circuit is adapted to produce said control signal in dependence of a current flowing through a high impedance load connected to said output terminals due to a high voltage developed there across.

15.     A cardiac stimulator comprising a high voltage generating circuit arrangement having a pair of output terminals, a step up transformer with a secondary winding which is connected to said output terminals and with a primary winding, a DC-supply source for supplying DC-energy to said primary winding, an electronic control device with a main current path and a control electrode for receiving a control voltage, said control device having its main current path connected in series with said primary winding and said DC-supply source; a current sensing circuit adapted for sensing output current flowing through a high impedance load connected to said output terminals for producing a control signal corresponding to sensed output current; and regulating means responsive to said control signal for applying a drive signal to said control electrode whereby the level of electric current flowing through said secondary winding is controlled.

16.    A cardiac stimulator according to claim 15 wherein said regulating means comprises a control amplifier having a first input for receiving said control signal and a second input for receiving a waveform signal determinative for the electric current flowing through said secondary winding.

17.    A cardiac stimulator comprising a high voltage generating circuit arrangement having a pair of output terminals, voltage generating means adapted to produce across said output terminals a high voltage of sufficient magnitude for causing an electric current to flow through a high impedance load connected to said output terminals, said electric current having a magnitude and waveform suitable for cardiac stimulation, control means which in response to control signals are effective to control said high voltage developed across said output terminals, and a sensing circuit being adapted to produce said control signal in dependence of the magnitude of the impedance of the load connected across said output terminals.

18.    A cardiac stimulator comprising a high voltage generating circuit arrangement having a pair of output terminals, a high voltage capacitor having its one terminal connected to one of said output terminals and its other terminal connected to a DC-supply source capable of charging said capacitor to a high voltage of sufficient magnitude for causing an electric current to flow through a high impedance load connected to said output terminals, said electric current having a magnitude and waveform suitable for cardiac stimulation, a controllable switching means having a control terminal and adapted for selectively discharging said capacitor in response to a control voltage applied to said control terminal, and a sensing circuit connected to one of said output terminals for producing a control signal for controlling the level of the output current which is caused to flow due to discharge of said capacitor through said load connected to said output terminals.

19.    A cardiac stimulator comprising a high voltage generating circuit arrangement having a pair of output terminals, a high voltage capacitor having its one terminal connected to one of said output terminals and having its other terminal connected to a DC supply source with a control terminal for receiving a control signal for selectively adjusting the DC voltage output level of said source, said source being capable of charging said capacitor to a high voltage of sufficient magnitude for causing an electric current to flow through a high impedance load connected to said output terminals, said electric current having a magnitude and waveform suitable for cardiac stimulation, a controllable switching means having a control terminal and adapted for selectively discharging said capacitor in response to a waveform signal applied to said control terminal, and a sensing circuit is connected with one of said output terminals for measuring magnitude of the impedance of said load connected to said output terminals for producing said control signal for application to said control terminal of said DC-supply source.

20.    In combination a cardiac stimulator according to any one of the foregoing claims, and an ECG processing circuit having a high input impedance, said high input impedance being connected to said output terminals of said high voltage generating circuit arrangement.

21.    In combination a cardiac stimulator according to any one of the foregoing claims, and an ECG processing circuit with a high input impedance, said high input impedance being connected to said output terminals of said high voltage generating circuit arrangement, said processing circuit being adapted to produce a R-wave-peak signal representative of peaks of a R-wave induced across said output terminals of said high voltage generating circuit arrangement, and a feedback circuit for applying said R-wave-peak signal to a control terminal of said high voltage generating circuit arrangement.

22.    A cardiac stimulator comprising a high voltage generating circuit arrangement having a pair of output terminals and a pair of input terminals, said high voltage generating circuit arrangement being adapted to produce across said output terminals a high voltage of sufficient magnitude for causing an electric current to flow through a high impedance load connected to said output terminals, said electric current having a magnitude and waveform suitable for cardiac stimulation, in response to a signal applied to said input terminals.

23.    A cardiac stimulator comprising a waveform signal generator for selectively producing a desired waveform signal and a high voltage generating circuit arrangement having a pair of output terminals and adapted to produce in response to said waveform signal, across said output terminals a high voltage of sufficient magnitude for causing an electric current to flow through a high impedance load connected to said output terminals, said electric current having a magnitude and waveform suitable for cardiac stimulation.

24.    A cardiac stimulator according to claims 22 or 23, wherein said waveform signal generator is adapted to produce said waveform signal with a repetition rate which is substantially linearly related to the setting of a variable resistor included in a rate determining network of said waveform signal generator, and a high voltage generating circuit arrangement having a pair of output terminals and adapted to produce in response to said waveform signal, across said output terminals a high voltage of sufficient magnitude for causing an electric current to flow through a high impedance load connected to said output terminals, said electric current having a magnitude and waveform suitable for cardiac stimulation.

25.    A cardiac stimulator according to claim 22, wherein a waveform signal generator of conventional design is connected to said input terminals.

26.    Tube section made of self sealing material and having at its opposite ends auxiliary connecting means for connecting said tube section into a catheter flow circuit, said tube section being intended for use in combination with a cardiac stimulator according to claim 4.

27.    Tube section having at its opposite ends auxiliary connecting means made integral therewith, said connecting means being adapted for connecting said tube section into a catheter flow circuit, said tube section being intended for use in combination with a cardiac stimulator according to claim 4.

28.    Tube section of self sealing material and having at its opposite ends auxiliary connecting means made integral therewith, said connecting means for connecting said tube section into a catheter flow circuit, said tube section being intended for use in combination with a cardiac stimulator according to claim 4.

29.    A cardiac stimulator comprising a high voltage generating circuit arrangement having a pair of output terminals, voltage generating means adapted to produce across said output terminals a high voltage of sufficient magnitude for causing an electric current to flow through a high impedance load connected to said output terminals, said electric current having a magnitude and waveform suitable for cardiac stimulation, wherein a capacitor is connected in series with one of said output terminals for inhibiting any DC current to flow through said high impedance load, and control means which in response to a control signal are effective to control said high voltage developed across said output terminals.

30. A cardiac stimulator according to claim 13, wherein said sensing circuit being further adapted to receive an auxiliary voltage representing any DC current flowing through said high impedance load circuit, and feedback means are provided which in response to said auxiliary voltage causes a DC current to flow through said high impedance load circuit by which said first mentioned DC current is compensated to substantially zero.

31. A cardiac stimulator according to claim 12, further comprising a voltage dependent element which presents a relatively low impedance when the high voltage developed across said output terminals exceeds a threshold value, said low impedance providing a substitute for a load normally to be connected to the output terminals, said element presents a relatively high impedance when said output voltage across said output terminals is lower than said threshold.

32. A cardiac stimulator according to claim 12, said control means comprising negative feedback means which in response to a voltage derived from said high voltage produced across said output terminals are further effective to limit the magnitude thereof to a predetermined value.

33. A cardiac stimulator according to one of the claims 12 through 32, wherein two electric resistors are connected in series relation in the electric current path between one output terminal of said high voltage generating circuit and the active catheter electrode.

0090467

1/5

FIG.6

FIG.7

FIG.8

FIG.9

0090467

FIG.1

FIG.2

FIG.3

FIG.4

FIG.5

FIG.10

FIG.11

FIG.12

3/5

0090467

FIG.13

FIG.14

0090467

FIG.15

FIG.16

FIG.17